# EUROPÄISCHE PATENTSCHRIFT

(11) **EP 3 176 156 B1**
(45) Veröffentlichungstag und Bekanntmachung des Hinweises auf die Patenterteilung: **12.06.2019**
(21) Anmeldenummer: 16204983.7
(22) Anmeldetag: 18.09.2012
(51) Int. Cl.: C07D 277/46, A61K 31/426, A61K 8/49, A61P 17/00, A61Q 19/02

(54) **ALKYLAMIDOTHIAZOLE, SIE ENTHALTENDE KOSMETISCHE ODER DERMATOLOGISCHE ZUBEREITUNGEN UND DEREN VERWENDUNG ZUR BEKÄMPFUNG UND PROPHYLAXE UNERWÜNSCHTER PIGMENTIERUNG DER HAUT**
ALKYLAMIDOTHIAZOLES, COSMETIC AND DERMATOLOGICAL PREPARATIONS CONTAINING THEM, AND THEIR USE FOR THE TREATMENT AND PROPHYLAXIS OF UNWANTED PIGMENTATION OF THE SKIN
ALKYLAMIDOTHIAZOLES, PRÉPARATIONS COSMÉTIQUES OU DERMATOLOGIQUES LES COMPRENANT, ET LEUR UTILISATION POUR LE TRAITEMENT ET LA PRÉVENTION DE LA PIGMENTATION INDÉSIRABLE DE LA PEAU

(30) Priorität: 23.09.2011 DE 102011083259
(43) Veröffentlichungstag der Anmeldung: 07.06.2017
(62) Teilanmeldung aus: 12762266.0
(73) Patentinhaber: Beiersdorf AG, 20253 Hamburg (DE)
(72) Erfinder: Kolbe, Ludger, 21255 Dohren (DE); Scherner, Cathrin, 22851 Norderstedt (DE); Breitkreutz, Sabrina, 20251 Hamburg (DE); Wöhrmann, Michael, 22851 Norderstedt (DE); Mann, Tobias, 22175 Hamburg (DE); Gerwat, Wolfram, 22850 Norderstedt (DE); Schläger, Torsten, 22297 Hamburg (DE)

(56) Entgegenhaltungen:
- EP-A1- 1 649 852
- WO-A2-2011/117034
- DATABASE CA [Online] CHEMICAL ABSTRACTS SERVICE, COLUMBUS, OHIO, US; 1998, SHRIVASTAVA, A. K. ET AL: "Syntheses of some 2-amino-4-(aryl/substituted aryl)thiazoles and their thiazolylamides as potential antifungal agents. II", XP002687088, gefunden im STN Database accession no. 1998:209541 & SHRIVASTAVA, A. K. ET AL: "Syntheses of some 2-amino-4-(aryl/substituted aryl)thiazoles and their thiazolylamides as potential antifungal agents. II", JOURNAL OF THE INSTITUTION OF CHEMISTS (INDIA) , 69(6), 167-168 CODEN: JOICA7; ISSN: 0020-3254, 1997,
- AGRAWAL R K ET AL: "Synthesis of some basic N-4-arylthiazolyl and n-4(substituted) arylthiazolylbutyramides as potential local anaesthetics", JOURNAL OF THE INDIAN CHEMICAL SOCIETY, THE INDIAN CHEMICAL SOCIETY, CALCUTTA; IN, Bd. 58, 1. Januar 1981 (1981-01-01), Seiten 787-788, XP009086881, ISSN: 0019-4522
- DATABASE CA [Online] CHEMICAL ABSTRACTS SERVICE, COLUMBUS, OHIO, US; 2010, PATTAN, S. R. ET AL: "Synthesis and evaluation of some new phenylthiazole derivatives for their anti-microbial activities", XP002687089, gefunden im STN Database accession no. 2010:919247 & PATTAN, S. R. ET AL: "Synthesis and evaluation of some new phenylthiazole derivatives for their anti-microbial activities", ASIAN JOURNAL OF RESEARCH IN CHEMISTRY , 2(3), 292-296 CODEN: AJRCBL; ISSN: 0974-4150 URL: HTTP://WWW.AJRCONLINE.ORG/PDF/AJRC_2_3_200 9_ABSTRACT.PDF, 2009,
- MORALES-BONILLA, PEDRO ET AL: "Preparation, antimicrobial activity, and toxicity of 2-amino-4-arylthiazole derivatives", HETEROATOM CHEMISTRY, CODEN: HETCE8; ISSN: 1042-7163, Bd. 17, Nr. 4, 2006, Seiten 254-260, XP002687090,
- DATABASE CA [Online] CHEMICAL ABSTRACTS SERVICE, COLUMBUS, OHIO, US; 2007, CHHABRIA, M. T. ET AL: "Synthesis and antifungal activity of some novel N-[4-(4-substituted phenyl)-1,3-thiazol-2-yl]-2-(1H-1,2,4-tria zol-1-yl)propionamides", XP002687091, gefunden im STN Database accession no. 2007:1311273 & CHHABRIA, M. T. ET AL: "Synthesis and antifungal activity of some novel N-[4-(4-substituted phenyl)-1,3-thiazol-2-yl]-2-(1H-1,2,4-tria zol-1-yl)propionamides", ACTA CIENCIA INDICA, CHEMISTRY , 33(1), 101-103 CODEN: ACICDV; ISSN: 0253-7338, 2007,
- GERMANAS ET AL: "Discovery of small-molecule inhibitors of tyrosinase", BIOORGANIC & MEDICINAL CHEMISTRY LETTERS, PERGAMON, ELSEVIER SCIENCE, GB, Bd. 17, Nr. 24, 12. Oktober 2007 (2007-10-12), Seiten 6871-6875, XP022339589, ISSN: 0960-894X, DOI: 10.1016/J.BMCL.2007.10.014

## Beschreibung

Die vorliegende Erfindung betrifft neue Alkylamidothiazole, kosmetische oder dermatologische Zubereitungen mit einem Gehalt an einem oder mehreren solcher Alkylamidothiazole und Alkylamidothiazole bzw. Zubereitungen, solche Alkylamidothiazole enthaltend, zur Bekämpfung und Prophylaxe unerwünschter Pigmentierung der Haut.

Für die Pigmentierung der Haut verantwortlich sind die Melanozyten, welche in der untersten Schicht der Epidermis, dem Stratum basale, neben den Basalzellen als -je nach Hauttyp entweder vereinzelt oder aber mehr oder weniger gehäuft auftretende - pigmentbildende Zellen vorzufinden sind.

Melanozyten enthalten als charakteristische Zellorganellen Melanosomen, in denen das Melanin gebildet wird. Unter anderem bei Anregung durch UV-Strahlung wird verstärkt Melanin gebildet. Dieses wird über die lebenden Schichten der Epidermis (Keratinozyten) letztlich in die Hornschicht (Corneozyten) transportiert und ruft eine mehr oder weniger ausgeprägte bräunliche bis braun-schwarze Hautfarbe hervor.

Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird. DHICA- und DHI-Melanin entstehen über die gemeinsamen Zwischenstufen Dopachinon und Dopachrom. Letzteres wird, teilweise unter Beteiligung weiterer Enzyme, entweder in Indol-5,6-Chinon-Carbonsäure oder in Indol-5,6-Chinon umgesetzt, woraus die beiden genannten Eumelanine entstehen.

Die Entstehung von Phäomelanin läuft unter anderem über die Zwischenprodukte Dopachinon und Cysteinyldopa. Gesteuert wird die Expression der Melanin-synthetisierenden Enzyme durch einen spezifischen Transkriptionsfaktor (microphthalmia- associated transcription factor, MITF). Neben den beschriebenen enzymatischen Prozessen der Melanin-Synthese sind in den Melanosomen noch weitere Proteine für die Melanogenese von Bedeutung. Eine wichtige Rolle scheint hier dem sogenannten p-Protein zuzukommen, wobei die exakte Funktion noch unklar ist.

Neben dem zuvor beschriebenen Prozeß der Melanin-Synthese in den Melanozyten, ist bei der Pigmentierung der Haut auch der Transfer der Melanosomen, deren Verbleib in der Epidermis sowie deren Abbau und der Abbau des Melanins von entscheidender Bedeutung. Es konnte gezeigt werden dass für den Transport der Melanosomen aus den Melanozyten in die Keratinozyten der PAR-2-Rezeptor bedeutsam ist (M. Seiberg et al., 2000, J. Cell. Sci., 113:3093-101).

Ferner haben Größe und Form der Melanosomen Einfluß auf ihre lichtstreuenden Eigenschaften und somit das farbliche Erscheinungsbild der Haut. So findet man bei Schwarzafrikanern verstärkt große spheroidale, einzeln vorliegende Melanosomen, während man bei Kaukasiern eher kleinere, in Gruppen vorkommende Melanosomen vorfindet.

Probleme mit Hyperpigmentierung der Haut haben vielfältige Ursachen bzw. sind Begleiterscheinungen vieler biologischer Vorgänge, z.B. UV-Strahlung (z.B. Sommersprossen, *Ephelides*), genetische Disposition, Fehlpigmentierung der Haut bei der Wundheilung bzw.-vernarbung (postinflammatorische Hyperpigmentierung) oder der Hautalterung (z.B. *Lentigines seniles*).

Nach entzündlichen Reaktionen reagiert das Pigmentierungssystem der Haut mit teilweise entgegengesetzten Reaktionen. Es kann sowohl zu postinflammatorischen Hyper- wie auch Hypopigmentierungen kommen. Postinflammatorische Hypomelanosen treten u. a. häufig in Verbindung mit Atopie, Lupus erythematosus und Psoriasis auf. Die unterschiedlichen Reaktionsformen des Pigmentierungssystems der menschlichen Haut in Folge entzündlicher Erscheinungen sind nur sehr unvollständig verstanden.

Probleme mit postinflammatorischer Hyperpigmentierung treten häufig bei dunkleren Hauttypen auf. Insbesondere bei männlichen Farbigen ist das Problem der *Pseudofollikulitis barbae* bekannt, das mit kosmetisch unerwünschten Fehlpigmentierung einhergeht bzw. diese nach sich zieht. Auch Formen von Melasma, welche insbesondere bei Frauen asiatischer Zugehörigkeit im Gesicht und im Dekolleté-Bereich auftreten, sowie verschiedene Formen der unregelmäßigen Pigmentierung der Haut werden zu den postinflammatorischen Hyperpigmentierungen gezählt. Ferner werden auch dunkle Augenringe als eine Form postinflammatorischen Hyperpigmentierungen angesehen, wobei die zugrunde liegende Entzündung meist subklinisch abläuft.

In vielen Fällen wird derartige postinflammatorische Fehlpigmentierung durch Einwirkung von Sonnenlicht (UV-Licht) noch verstärkt, ohne dass es zu einer UV-induzierten Entzündung (Sonnenbrand) kommt.

Es sind Wirkstoffe und Zubereitungen bekannt, welche der Hautpigmentierung entgegenwirken. Im praktischen Gebrauch sind im wesentlichen Präparate auf der Grundlage von Hydrochinon, welche aber einesteils erst nach mehrwöchiger Anwendung ihre Wirkung zeigen, deren übertrieben lange Anwendung andererseits aus toxikologischen Gründen bedenklich ist. Von Albert Kligman et al. wurde eine sogenannte "Triformula" entwickelt, die eine Kombination aus 0.1% Tretinoin, 5.0% Hydrochinon, 0.1% Dexamethason darstellt (A. Kligman, 1975, Arch. Dermatol., 111:40-48). Allerdings ist auch diese Formulierung wegen möglicher irreversibler Veränderungen im Pigmentierungssystem der Haut sehr umstritten.

Ferner finden hautschälende Methoden (chemische und mechanische "Peelings") Anwendung, die jedoch häufig entzündliche Reaktionen nach sich ziehen und aufgrund danach eintretender postinflammatorischer Hyperpigmentierungen sogar zu stärkerer statt verminderter Pigmentierung führen können. All diese gängigen Verfahren, die auch zur Behandlung von postinflammatorischen Hypergigmentierungen angewendet werden, zeichnen sich durch entscheidende Nebenwirkungen aus.

Weiterhin sind diverse andere Substanzen bekannt, für die eine hautaufhellende Wirksamkeit beschrieben wird. U.a. zu nennen sind hier Hexadecen-1,16-dicarbonsäure, Kojic-Säure und Derivate, Arbutin, Ascorbinsäure und Derivate, Flavonoide, Ellagsäure und Derivate, Tranexamsäure und verschiedene Resorcinol-Derivate, wie z.B. 4-n-Butylresorcin, 4-n-Hexylresorcin und 4-(1-phenylethyl)benzen-1,3-diol.

J.M. Ready beschreibt in einer Publikation (Bioorganic & Medicinal Chemistry Letter 17 (2007) 6871-6875 die Wirkung von u.a. substituierten Thiazol-Derivaten zur Inhibition der Mushroom tyrosinase.

In der Patentanmeldung der Firma Shiseido (WO 2009/099195) werden substituierte Thiazolamine bzw. Hydrothiazolamine zur Hautaufhellung beschrieben. EP1649852 offenbart heterozyklische Verbindungen zur Behandlung von unerwünschter Pigmentierung.

Die im oben genannten Stand der Technik beschriebenen Substanzen weisen sich durch eine moderate Wirksamkeit und/oder eine schlechte galenische Stabilität aus.

Augenringe können ebenfalls als Folgen einer Pigmentierungsstörung entstehen, wobei sie ferner auch als Reaktion auf allgemeinen Stress, wie z.B. wenig Schlaf oder schlicht durch Überanstrengung der Augen erscheinen. Bei jüngeren Menschen verschwinden die Symptome nach ausreichender Nachtruhe wieder, über längere Zeiträume jedoch kann der Zustand chronisch und für die betroffenen Personen sehr störend werden. Auch gegen solche Hauterscheinungen mangelt es an genügend erfolgversprechenden Wirkstoffen und Behandlungsmöglichkeiten. Strukturen, welche nicht unter die Ansprüche fallen, dienen dem Verständnis, bilden jedoch nicht Teil der Erfindung.

Ziel der nachfolgenden Erfindung war es also, dem nachteiligen Stand der Technik Abhilfe zu verschaffen.

Beschrieben sind Alkylamidothiazole der allgemeinen Formel 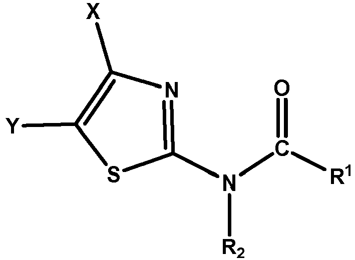 bei welcher
R¹, R², X und Y können unterschiedlich, teilweise gleich oder völlig gleich sein und unabhängig voneinander bedeuten können:
**R₁** = -C₁-C₂₄-Alkyl (linear und verzweigt), -C₁-C₂₄-Alkenyl (linear und verzweigt), -C₁-C₈-Cycloalkyl, -C₁-C₈-Cycloalkyl-Alkylhydroxy, -C₁-C₂₄ Alkylhydroxy (linear und verzweigt), -C₁-C₂₄ Alkylamin (linear und verzweigt), -C₁-C₂₄-Alkylaryl (linear und verzweigt), -C₁-C₂₄-Alkylaryl-Alkyl-Hydroxy (linear und verzweigt), -C₁-C₂₄-Alkylheteroaryl (linear und verzweigt), -C₁-C₂₄-Alkyl-O-C₁-C₂₄-Alkyl (linear und verzweigt), -C₁-C₂₄ Alky-Morpholino, -C₁-C₂₄ Alky-Piperidino, -C₁-C₂₄ Alky-Piperazino, -C₁-C₂₄ Alky-Piperazino-N-Alkyl bedeutet,
**R₂** = H, -C₁-C₂₄-Alkyl (linear und verzweigt), -C₁-C₂₄-Alkenyl (linear und verzweigt), -C₁-C₈-Cycloalkyl, -C₁-C₂₄-Hydroxyalkyl (linear und verzweigt), -C₁-C₂₄-Alkylaryl (linear und verzweigt), -C₁-C₂₄-Alkylheteroaryl (linear und verzweigt), bedeutet,
**X =** -H, -C₁-C₂₄-Alkyl (linear und verzweigt), -C₁-C₂₄-Alkenyl (linear und verzweigt), -C₁-C₈-CycloAlkyl, -C₁-C₂₄-Aryl (ggfs. einfach oder mehrfach substituiert mit -OH, -F, -Cl, -Br, -I,-OMe, -NH₂, -CN), -C₁-C₂₄-Heteroaryl (ggfs. einfach oder mehrfach substituiert mit -OH, -F,-Cl, -Br, -I, -OMe, -NH₂, -CN), -C₁-C₂₄-Alkylaryl (linear und verzweigt), -C₁-C₂₄-Alkylheteroaryl (linear und verzweigt), -Aryl (ggfs. einfach oder mehrfach substituiert mit -OH, -F, -Cl,-Br, -I, -OMe, -NH₂, -CN), -phenyl, -2,4-dihydroxyphenyl, -2,3-dihydroxyphenyl, -2,4-Dimethoxyphenyl, -2,3-Dimethoxyphenyl bedeutet,
**Y =** H, -C₁-C₂₄-Alkyl (linear und verzweigt), -C₁-C₂₄-Alkenyl (linear und verzweigt), -C₁-C₈-Cycloalkyl, -C₁-C₂₄-Aryl, -C₁-C₂₄-Heteroaryl, -C₁-C₂₄-Alkylaryl (linear und verzweigt), -C₁-C₂₄-Alkylheteroaryl (linear und verzweigt), -Aryl, -phenyl, -2,4-dihydroxyphenyl, -2,3-dihydroxyphenyl, -2,4-Dimethoxyphenyl, -2,3-Dimethoxyphenyl, -COO-Alkyl, -COO-Alkenyl,-COO-Cylcloalkyl, -COO-Aryl, -COO-Heteroaryl , bedeutet,
und **X, Y** gegebenenfalls auch **=** kondensierter Aromat bedeuten können,
wobei **X** und **Y** untereinander aromatische oder aliphatische homo- oder heterozyklische Ringsysteme mit bis n ringbildenden Atomen ausbilden können, und wobei die Zahl n Werte von 5 bis 8 annehmen kann, und die jeweiligen Ringsysteme wiederum mit bis zu n - 1 Alkylgruppen, Hydroxylgruppen, Carboxylgruppen, Aminogruppen, Nitrilfunktionen, Schwefelhaltige Substituenten, Estergruppen und/oder Ethergruppen substituiert sein können.

Die genannten Thiazole können sowohl als freie Base wie auch als Salz vorliegen: z.B. als Fluorid, Chlorid, Bromid, lodid, Sulfat, Carbonat, Ascorbat, Acetat oder Phoshat. Im Besonderen als Halogensalze, wie z.B. Chlorid und Bromid.

Weiterhin besteht eine vorteilhafte Verwirklichung der vorliegenden Erfindung in kosmetischen oder dermatologischen Zubereitungen mit einem wirksamen Gehalt an einem oder mehreren vorbenannten Alkylamidothiazolen.

Erfindungsgemäß sind Alkylamidothiazole zur Behandlung und/oder Prophylaxe unerwünschter Hautpigmentierung.

Dabei können Behandlung und/oder Prophylaxe unerwünschter Hautpigmentierung sowohl im kosmetischen wie im pharmazeutischen Rahmen erfolgen.

Dabei, wird die pharmazeutische (oder dermatologische) Behandlung in erster Linie bei krankhaften Hautzuständen verstanden, wogegen die kosmetische Behandlung und/oder Prophylaxe unerwünschter Hautpigmentierung in erster Linie die gesunde Haut betrifft.

Beschrieben wird **X** gewählt aus der Gruppe der substituierten Phenyle, wobei die Substituenten (Z) gewählt werden können aus der Gruppe -H, -OH, -F, -Cl, -Br, -I, -OMe, -NH₂, -CN, Acetyl und gleich oder unterschiedlich sein können.

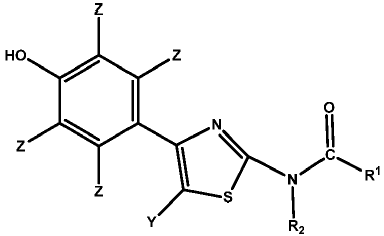

Beschrieben wird X aus der Gruppe der mit einer oder mehreren Hydroxygruppen substituierten Phenylgruppen wobei der Substituent (Z) gewählt werden kann aus der Gruppe -H, -OH, -F, -Cl, -Br, -I, -OMe, -NH₂, -CN, Acetyl und die folgende generische Struktur bevorzugt wird, bei welcher Y, R¹ und R² die vorstehend definierten Eigenschaften haben können. 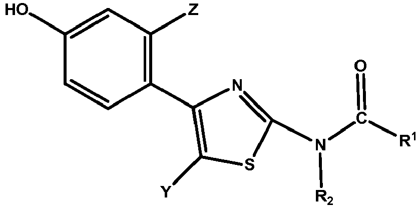 Beschrieben werden Verbindungen, bei welchen X= 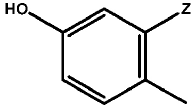
**Y** = H
**R₁ =** -C₁-C₂₄-Alkyl (linear und verzweigt), -C₁-C₂₄-Alkenyl (linear und verzweigt), -C₁-C₈-Cycloalkyl, -C₁-C₈-Cycloalkyl-Alkylhydroxy, -C₁-C₂₄ Alkylhydroxy (linear und verzweigt), -C₁-C₂₄ Alkylamin (linear und verzweigt), -C₁-C₂₄-Alkylaryl (linear und verzweigt), -C₁-C₂₄-Alkylaryl-Alkyl-Hydroxy (linear und verzweigt), -C₁-C₂₄-Alkylheteroaryl (linear und verzweigt), -C₁-C₂₄-Alkyl-O-C₁-C₂₄-Alkyl (linear und verzweigt), -C₁-C₂₄ Alky-Morpholino, -C₁-C₂₄ Alky-Piperidino, -C₁-C₂₄ Alky-Piperazino, -C₁-C₂₄ Alky-Piperazino-N-Alkyl bedeutet,
**R₂ =** H, -C₁-C₂₄-Alkyl (linear und verzweigt).
**Z =** -H, -OH, -F, -Cl, -Br, -I, -OMe, -NH₂, -CN, Acetyl. Erfindungsgemäss sind solche Verbindungen, bei welchen **X =** 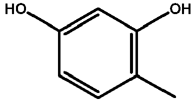
**Y** = H
**R₁ =** -C₁-C₂₄-Alkyl (linear und verzweigt), -C₁-C₂₄-Alkenyl (linear und verzweigt), -C₁-C₈-Cycloalkyl, -C₁-C₈-Cycloalkyl-Alkylhydroxy, -C₁-C₂₄ Alkylhydroxy (linear und verzweigt), -C₁-C24 Alkylamin (linear und verzweigt), -C₁-C₂₄-Alkylaryl-Alkyl-Hydroxy (linear und verzweigt), -C₁-C₂₄-Alkylheteroaryl (linear und verzweigt), -C₁-C₂₄-Alkyl-O-C₁-C₂₄-Alkyl (linear und verzweigt), -C₁-C₂₄ Alky-Morpholino, -C₁-C₂₄ Alky-Piperidino, -C₁-C₂₄ Alky-Piperazino, -C₁-C₂₄ Alky-Piperazino-N-Alkyl bedeutet,
**R₂ =** H.

Die Verbindungen 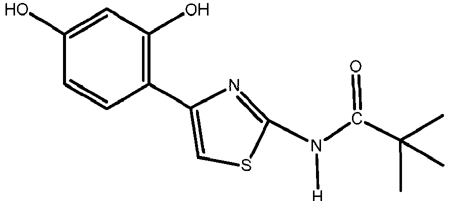 *N*-(4-(2,4-dihydroxyphenyl)thiazol-2-yl)pivalamide 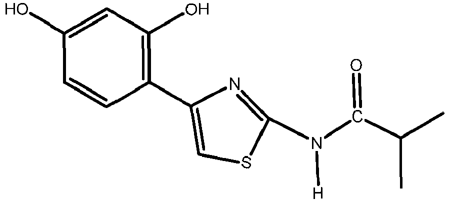 *N*-(4-(2,4-dihydroxyphenyl)thiazo1-2-y1)isobutyramide 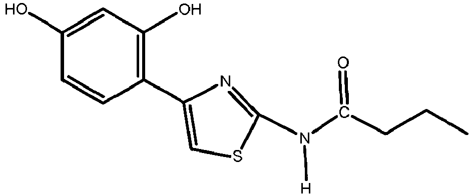 *N*-(4-(2,4-dihydroxyphenyl)thiazol-2-yl)butyramide 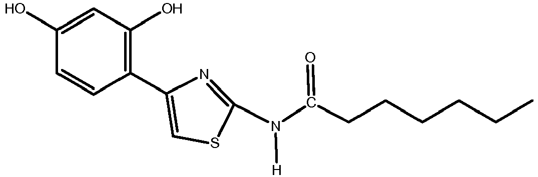 *N*-(4-(2,4-dihydroxyphenyl)thiazol-2-yl)heptanamide 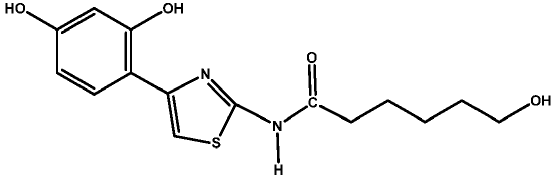 *N*-(4-(2,4-dihydroxyphenyl)thiazol-2-yl)-6-hydroxyhexanamide 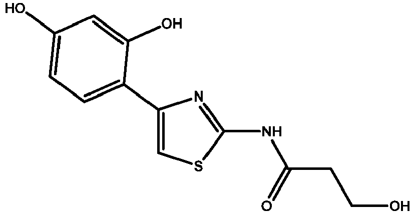 *N*-(4-(2,4-dihydroxyphenyl)thiazol-2-yl)-3-hydroxypropanamide 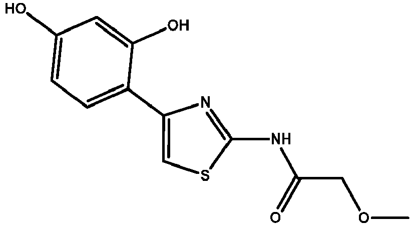 *N*-(4-(2,4-dihydroxyphenyl)thiazol-2-yl)-2-methoxyacetamide 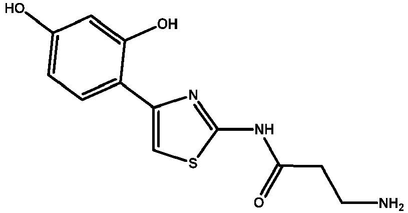 3-amino-*N*-(4-(2,4-dihydroxyphenyl)thiazo1-2-yl)propanamide 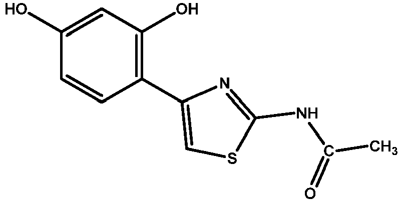 *N*-(4-(2,4-dihydroxyphenyl)thiazol-2-yl)acetamide 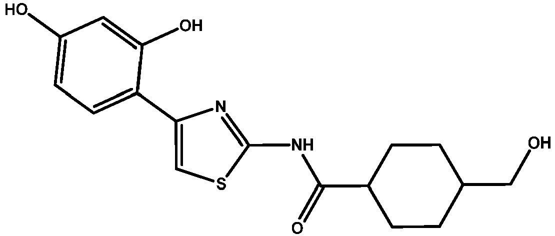 *N*-(4-(2,4-dihydroxyphenyl)thiazol-2-yl)-4-(hydroxymethyl)cyclohexanecarboxamide 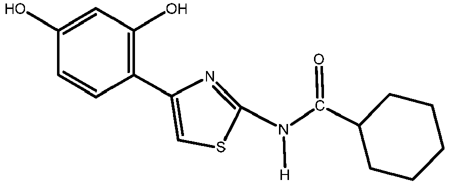 *N*-(4-(2,4-dihydroxyphenyl)thiazol-2-yl)cyclohexanecarboxamide
und 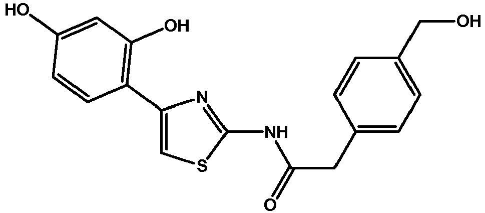 *N*-(4-(2,4-dihydroxyphenyl)thiazol-2-yl)-2-(4-(hydroxymethyl)phenyl)acetamide sind die erfindungsgemäß bevorzugten.
Überraschenderweise konnte gezeigt werden, dass die erfindungsgemäßen Alkylamidothiazole im Vergleich zu den entsprechenden Alkylaminothiazolen eine höhere galenische Stabilität aufweisen und/oder eine gesteigerte Wirksamkeit.

Siehe Tabelle 1 und Tabelle 2.

### Methodenbeschreibung der Stabilitätsuntersuchungen:

Die Amine und Amide wurden zur Einarbeitung in die Formulierungen in Butylenglykol - ggf. unter Erwärmung - gelöst und vor dem 1. Homogenisieren bei ca. 65 °C zur Emulsion gegeben. Alle Wirkstoffe wurden in den Versuchsansätzen in einer Konzentration von 0.1% eingearbeitet.

Die Emulsion wurde für die Lagertests in 20ml Glasvials abgefüllt und unter verschiedenen Standardbedingungen (Raumtemperatur, Licht und 40°C) gelagert.

Für die Stabilitätsuntersuchungen wurden die gelagerten Proben nach 14 Tagen analytisch untersucht.

### Analytik/Wiederfindung:

Die zu vermessenen Proben wurden in einem Methanol/Wasser-Gemisch [70:30] extrahiert und mittels HPLC-DAD bestimmt. Die Bestimmung erfolgte mittels externer Standardkalibrierung [Referenzteil]. Die Auswertung erfolgte bei 296 nm.

### Gerät:

HPLC: Agilent 1100
Säule: Phenomenex Synergi MAX-RP, 50 x 2 mm i. d. [2.5 µm]
Lösungsmittel: Gradient Acetonitril/Wasser mit 0.1% Phosphorsäure
Flussrate: 0,3 mL/min

| Gradient: | Zeit [min] | H2O [%] | MeCN [%] |
|---|---|---|---|
| | | 0,1% H3PO4 | |
| | 0,0 | 90,0 | 10,0 |
| | 10,0 | 10,0 | 90,0 |
| | 12,0 | 10,0 | 90,0 |
| | 13,0 | 90,0 | 10,0 |
| | 20,0 | 90,0 | 10,0 |

Injektionsvolumen: 3 µL

Als Referenz dienten die jeweils eingesetzten Rohstoffe.

| **Tabelle 1:** | | |
|---|---|---|
| **Prüfmuster** | **Wiederfindung [%]** | |
| **Formel 1** - mit N-(4-(2,4-Dihydroxyphenyl)thiazol-2-yl)-isobutyramid | 100 | Amid |
| **Formel 2** - mit 4-(2-Isopropylamino)thiazol-4-yl)benzene-1,3-diol | 70 | Amin |
| **Formel 3** - mit 4-(2-(tert-Butylamino)thiazol-4-yl)benzen-1,3-diol | 93 | Amin |
| **Formel 4** - mit N-(4-(2,4-Dihydroxyphenyl)thiazol-2-yl)-pivalamid | 100 | Amid |
| **Formel 5** - mit N-(4-(2,4-Dihydroxyphenyl)thiazol-2-yl)-butyramid | 100 | Amid |
| **Formel 6** - mit 4-(2-propylamino)thiazol-4-yl)benzen-1,3-diol | 84 | Amin |
| **Formel 7** - mit N-(4-(2,4-Dihydroxyphenyl)thiazol-2-yl)-cyclohexancarboxamid | 99 | Amid |
| **Formel 8** - mit 4-(2-(Cyclohexylamino)thiazol-4-yl)benzen-1,3-diol | 90 | Amin |
| **Formel 9** - mit N-(4-(2,4-Dihydroxyphenyl)thiazol-2-yl)-heptanamid | 100 | Amid |
| **Formel 10 -** mit 4-(2-(Hexylamino)thiazol-4-yl)benzen-1,3-diol | 86 | Amin |

### Methodenbeschreibung der Wirksamkeitsuntersuchungen:

Die Wirksamkeit der Thiazole wurde mit einem Enzymtest belegt, in der Umsatz von L-DOPA zu L-Dopachinon durch eine humane Tyrosinase gemessen wurde. Bei dieser literaturbekannten Methode (Winder, A.J. and Harris, H., New assays for the tyrosine hydroxylase and dopa oxidase activities of tyrosinase. Eur. J. Biochem. (1991), 198, 317-26) wird das Reaktionsprodukt L-Dopaquinone mit MBTH (3-methyl-2-benzothiazoline hydrazone) zu einer pinkfarbenen Substanz umgesetzt, deren Zunahme über die Zeit durch Absorption bei 490 nm gemessen wird. In Tabelle ein sind Wirksamkeitsdaten für einige der beanspruchten Substanzen beispielhaft dargestellt. Daraus lässt sich schließen, dass die erfindungsgemä-ßen Substanzen äußerst effektive pigmentierungs-inhibierende Substanzen sind.

| **Tabelle 2: Inhibition der Tyrosinaseaktivität durch Thiazole** | | |
|---|---|---|
| *Substanz* | *Inhibition (% der Kontrolle)* | *Konzentration* |
| N-(4-(2,4-Dihydroxyphenyl)thiazol-2-yl)-isobutyramid | 94 | 10 µg/mL |
| 4-(2-Isopropylamino)thiazol-4-yl)benzene-1,3-diol | 91 | 10 µg/mL |
| N-(4-(2,4-Dihydroxyphenyl)thiazol-2-yl)-heptanamid | 66 | 10 µg/mL |
| 4-(2-(Hexylamino)thiazol-4-yl)benzen-1,3-diol | 41 | 10 µg/mL |
| 4-(2-(tert-Butylamino)thiazol-4-yl)benzen-1,3-diol | 62 | 10 µg/mL |
| N-(4-(2,4-Dihydroxyphenyl)thiazol-2-yl)-pivalamid | 96 | 10 µg/mL |
| N-(4-(2,4-Dihydroxyphenyl)thiazol-2-yl)-butyramid | 95 | 10 µg/mL |
| 4-(2-propylamino)thiazol-4-yl)benzen-1,3-diol | 88 | 10 µg/mL |
| N-(4-(2,4-Dihydroxyphenyl)thiazol-2-yl)-cyclohexancarboxamid | 92 | 10 µg/mL |
| 4-(2-(Cyclohexylamino)thiazol-4-yl)benzen-1,3-diol | 70 | 10 µg/mL |
| N-(4-(2,4-Dihydroxyphenyl)thiazol-2-yl)-4-(Hydroxymethyl)cyclohexancarboxamid | 94 | 10 µg/mL |
| 4-(2-((4-(hydroxymethyl)-phenyl)amino)-thiazol-4-yl)benzen-1,3-diol | 93 | 10 µg/mL |
| N-(4-(2,4-dihydroxyphenyl)thiazol-2-yl)-2-(4-(hydroxymethyl)phenyl)acetamid | 88 | 10 µg/mL |

### Synthesevorschriften exemplarisch ausgewählter Alkylamidothiazole:

### 2-Brom-2',4'-bis-methoxycarbonyloxy-acetophenon:

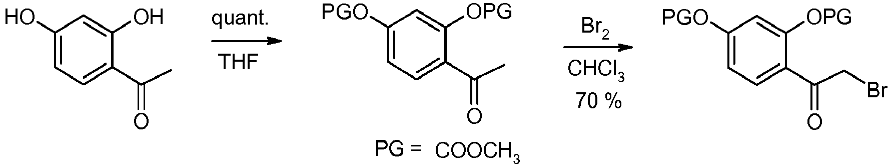

### Mitchell, David; Doecke, Christopher W.; Hay, Lynne A.; Koenig, Thomas M.; Wirth, David D. Tetrahedron Letters, 1995

Eine Lösung von 60g (369 mmol) 2,4-Dihydroxyacetophenon and 186 ml Triethylamin in 900 ml Tetrahydrofuran wurde auf 0°C gekühlt und 93 ml Chlorameisensäuremethylester in 400 ml Tetrahydrofuran langsam zugetropft. Es bildet sich ein weißer Niederschlag. Nach 3 Stunden Rühren bei Raumtemperatur ist die Reaktion abgeschlossen (DC-Kontrolle). Der Niederschlag wurde abgesaugt und mit reichlich Tetrahydrofuran gewaschen. Das Filtrat wurde zur Trockne einrotiert, in Ethylacetat aufgenommen mit 1N HCl und NaCl-Lösung (sat.) gewaschen und über Magnesiumsulfat getrocknet, vom Magnesiumsulfat filtriert und das Ethylacetat am Rotationsverdampfer eingeengt. Es wurden 105 g von 2,4-Bis-methoxycarbonyloxy-acetophenon erhalten. ¹H NMR (DMSO-D₆): 8.05 (d, 1H), 7.38 (d, 1H), 7.36 (s, 1H), 3.86 (d, 6H). Das Produkt wurde ohne weitere Reinigung eingesetzt. Zu der Lösung von 105 g 2,4-Bis-methoxycarbonyloxy-acetophenon in Chloroform (1000 ml) wurden 63g (392 mmol) Brom in 450 ml Chloroform innerhalb von 3 h zugetropft. Danach wurde die Reaktion noch 15 min. bei Raumtemperatur gerührt, Das Lösungsmittel wurde einrotiert. Der Rückstand wurde in Ethylacetat/n-Hexan verrührt, der entstandene Niederschlag wurde abgesaugt. Umkristallisation aus Ethylacetat/n-Hexan lieferten 100 g 2-Brom-2',4'- bis-methoxycarbonyloxy-acetophenon. ¹H NMR (DMSO-D₆): 8.11 (d, 1H), 7.42 (m, 2H), 4. 87 (s, 2H), 3.87 (s, 3H), 3.85 (s, 3H) ppm; m.p. 73-74°C.

### N-(4-(2,4-Dihydroxyphenyl)thiazol-2-yl)-pivalamid:

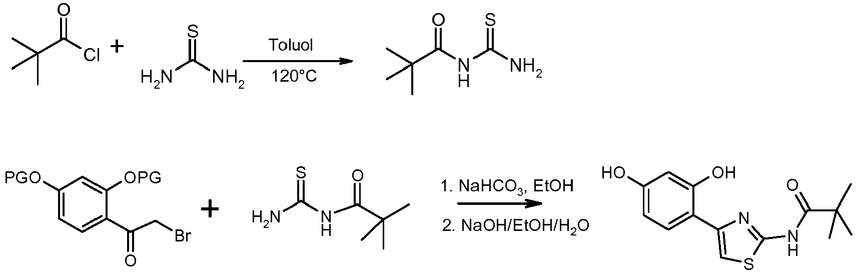

126 g (1.66 mmol) Thioharnstoff wurden in Toluol (1000 ml) vorlegt und 100 g (829 mmol) Pivaloylchlorid zugetropft. Die Reaktionslösung wurde 3 Stunden unter Rückfluss gekocht, wobei 2 Phasen entstehen. Die obere Phase wurde abdekantiert und abgekühlt. Die ausgefallenen farblosen Nadeln wurden abgesaugt und mit Cyclohexan gewaschen und im Vakuum getrocknet. Ausbeute: 64 g. ¹H NMR (DMSO-D₆): 10.27 (s, 1H), 9.74 (s, 1H), 9.40 (s, 1H), 1.19 (s, 9H) ppm.

107.7 g (310 mmol) 2-Brom-2',4'- bis-methoxycarbonyloxy-acetophenon wurden mit 49.7 g (13.6 mmol) N-Pivaloylthioharnstoff und 39.2 g (466 mmol) NaHCO₃ in 1.21 Ethanol unter Rückfluss für 0.5h gekocht. Die Reaktionslösung wurde abgekühlt und mit 50.6 g (1.27 mol) NaOH in 250 ml Wasser versetzt. Nach 30 Min. Rühren bei Raumtemperatur wurde die Reaktionslösung mit 300 ml Wasser aufgenommen und mit 2N HCl neutralisiert. Der entstandene Niederschlag wurde abfiltriert und aus Ethanol/Wasser umkristallisiert. Es wurden 80 g Thiazol erhalten. ¹H NMR (DMSO-D₆): 11.77 (bs, 1H), 11.02 (bs, 1H), 9.47 (bs, 2H), 7.65 (d, 1H), 7.39 (s, 1H), 6.30 (s, 1H), 6.28 (d, 1H), 1.27 (s, 9H) ppm; m.p. 257-259°C.

### N-(4-(2,4-Dihydroxyphenyl)thiazol-2-yl)-isobutyramid:

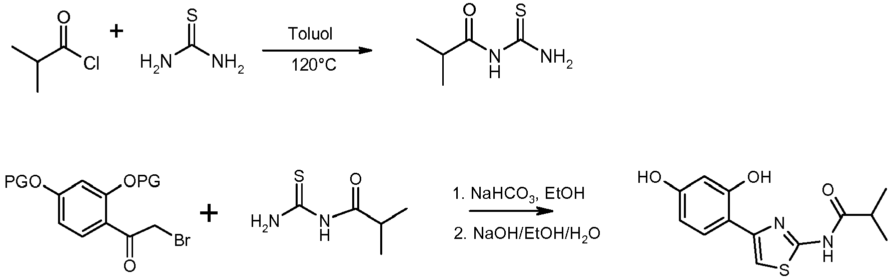

114 g (1.5 mol) Thioharnstoff wurden in Toluol (800 ml) vorgelegt und 80 g (0.75 mol) Isobutyrylchlorid zugetropft. Die Reaktionslösung wurde 3 Stunden unter Rückfluss gekocht, wobei 2 Phasen entstehen. Die obere Phase wurde abdekantiert und abgekühlt. Die ausgefallenen weißen Kristalle wurden abgesaugt und mit Toluol gewaschen und im Vakuum getrocknet. Ausbeute: 62 g. ¹H NMR (DMSO-D₆): 11.03 (bs, 1H), 9.66 (bs, 1H), 9.35 (bs, 1H), 2.72 (m, 1H), 1.03 (2, 6H) ppm;
89 g (260 mmol) 2-Brom-2',4'- bis-methoxycarbonyloxy-acetophenon wurden mit 37.5 g (260 mmol) N-Isobutyrylthioharnstoff und 32 g (380 mmol) NaHCO₃ in 1000 ml Ethanol unter Rückfluss für 0.5h gekocht. Die Reaktionslösung wurde abgekühlt und mit 41 g (0.93 mol) NaOH in 250 ml Wasser versetzt. Nach 30 Min. Rühren bei Raumtemperatur wurde die Reaktionslösung mit 300 ml Wasser aufgenommen und mit 2N HCl auf pH=3 eingestellt. Der entstandene Niederschlag wurde abfiltriert und aus Ethanol/Wasser umkristallisiert. Es wurden 56 g Thiazol erhalten. ¹H NMR (DMSO-D₆): 12.16 (bs, 1H), 10.88 (bs, 1H), 9.47 (bs, 1H), 7.65 (m, 1H), 7.41 (s, 1H), 6.32 (m, 2H), 2.75 (m, 1H), 1.14 (d, 6H) ppm. m.p.: 243-245°C.

### N-(4-(2,4-Dihydroxyphenyl)thiazol-2-yl)-butyramid:

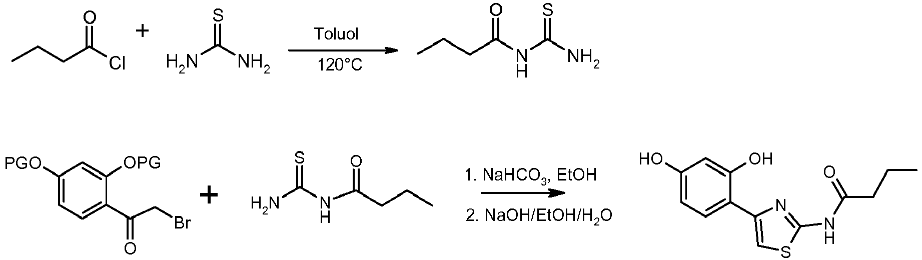

143 g (1.88 mol) Thioharnstoff wurden in Toluol (1000 ml) vorgelegt und 100 g (0.93 mol) n-Butyrylchlorid zugetropft. Die Reaktionslösung wurde 3 Stunden unter Rückfluss gekocht, wobei 2 Phasen entstehen. Die obere Phase wurde abdekantiert und abgekühlt. Die ausgefallenen leicht gelblichen Kristalle wurden abgesaugt und mit Toluol gewaschen und im Vakuum getrocknet. Ausbeute: 88 g. ¹H NMR (DMSO-D₆): 11.03 (bs, 1H), 9.65 (bs, 1H), 9.33 (bs, 1H), 2.33 (t, 2H), 1.53 (m, 2H), 0.86 (t, 3H) ppm; m.p.: 115-188°C
92 g (265 mmol) 2-Brom-2',4'- bis-methoxycarbonyloxy-acetophenon wurden mit 38.75 g (265 mmol) N-Butyrylthioharnstoff und 34 g (397 mmol) NaHCO₃ in 900 ml Ethanol unter Rückfluss für 0.5h gekocht. Die Reaktionslösung wurde abgekühlt und mit 37 g (0.93 mol) NaOH in 300 ml Wasser versetzt. Nach 30 Min. Rühren bei Raumtemperatur wurde die Reaktionslösung mit 300 ml Wasser aufgenommen und mit 2N HCl neutralisiert. Der entstandene Niederschlag wurde abfiltriert und aus Ethanol/Wasser umkristallisiert. Es wurden 67 g Thiazol erhalten. ¹H NMR (DMSO-D₆): 12.18 (bs, 1H), 10.89 (bs, 1H), 9.48 (bs, 1H), 7.65 (1 arom. H), 7.40 (s, 1H), 6.31 (2 arom. H), 2.43 (t, 2H), 1.64 (m, 2H), 0.91 (t, 3H)ppm. m.p.: 227-229°C.

### N-(4-(2,4-Dihydroxyphenyl)thiazol-2-yl)-acetamid:

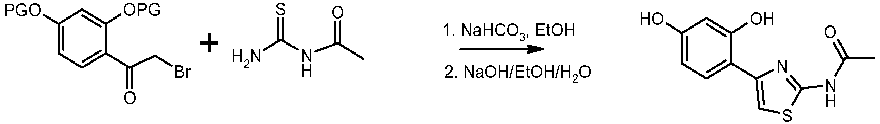

4.71 g (13.6 mmol) von 2-Brom-2',4'- bis-methoxycarbonyloxy-acetophenon wurden mit 1.61 g (13.6 mmol) N-Acetylthioharnstoff und 1.72 g (20.4 mmol) NaHCO₃ in 45 ml Ethanol 0.5h unter Rückfluss gekocht. Die Reaktionslösung wurde abgekühlt und mit 2.0 g (50 mmol) NaOH in 20 ml Wasser versetzt. Nach 20 Min. Rühren bei 0°C wurde die Reaktionslösung mit 30 ml Wasser aufgenommen und mit halbkonz. HCl neutralisiert. Der entstandene Niederschlag wurde abfiltriert und aus Ethanol/Wasser umkristallisiert. Es wurden 2.73g Produkt erhalten. ¹H NMR (DMSO-D₆): 12.20 (b, 1H), 10.85 (s, 1H), 9.46 (s, 1H), 7.64 (m, 1H), 7.38 (s, 1H), 6.28 (m, 2H), 2.15 (s, 3H) ppm; m.p. 264-264°C.

### N-(4-(2,4-Dihydroxy,phenyl)thiazol-2-yl)-4-(Hydroxymethyl)cyclohexancarboxamid:

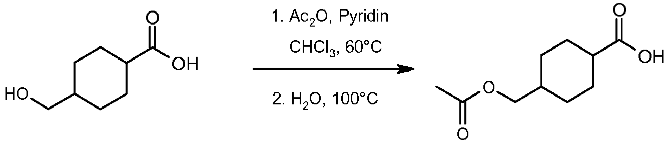

### Durchführung analog Literatur.

BANYU Pharmaceutical Co., Ltd., EP2072519 A1, 2009
Ausbeute: 96%, ¹H NMR (DMSO-D₆): 12.03 (bs, 1H), 3.85, 3.82 (2 x d, 2H), 2.50, 2.47 (2 x m, 1H), 2.00 (s, 3H), 0.95-1.90 (m, 9H) ppm; 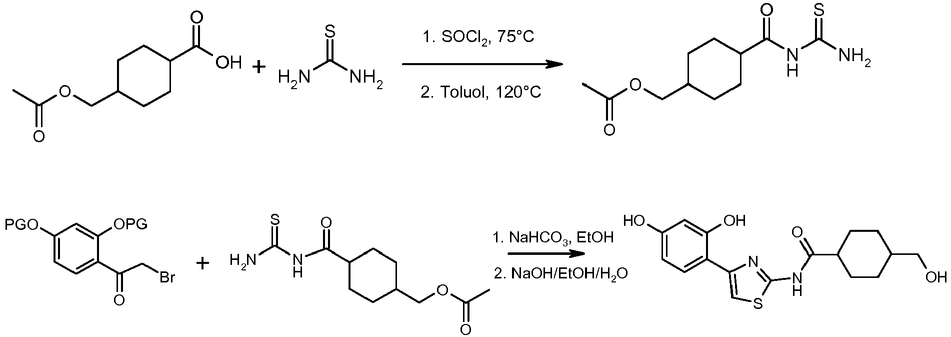 95 g (0.47 mol) 4-Acetoxymethylcyclohexancarbonsäure wurden in 350 ml Thionylchlorid 2h unter Rückfluss erhitzt. Nach Entfernen des überschüssigen Thionylchlorids im Vakuum wurde der Rückstand in 1l Toluol aufgenommen und 71 g (0.94 mol) Thioharnstoff zugegeben. Die Reaktionslösung wurde 3 Stunden unter Rückfluss gekocht und anschließend heiß filtriert. Nach Abkühlen der Mutterlauge wurden die entstandenen weißen Kristalle abgesaugt, mit Toluol gewaschen und im Vakuum getrocknet. Ausbeute: 59 g. ¹H NMR (DMSO-D₆): 11.03, 10.97 (2 x s, 1H), 9.64 (bs, 1H), 9.35 (bs, 1H), 3.93, 3.82 (2 x d, 2H), 2.61, 2.42 (2 x m, 1H), 2.00 (s, 3H), 1.60 (m, 8H), 1.35, 0.94 (2 x m, 1H) ppm;
79 g (228 mmol) 2-Brom-2',4'- bis-methoxycarbonyloxy-acetophenon wurden mit 59 g (228 mmol) N-(4-Acetoxymethylcyclohexylcarbonyl)thioharnstoff und 29 g (340 mmol) NaHCO₃ in 1000 ml Ethanol unter Rückfluss für 0.5h gekocht. Die Reaktionslösung wurde abgekühlt und mit 73 g (1.8 mol) NaOH in 300 ml Wasser versetzt. Nach 30 Min. Rühren bei Raumtemperatur wurde die Reaktionslösung mit 300 ml Wasser aufgenommen und mit 2N HCl auf pH=3 eingestellt. Der entstandene Niederschlag wurde abfiltriert und aus Ethanol/Wasser umkristallisiert. Es wurden 47 g Thiazol erhalten. ¹H NMR (DMSO-D₆): 12.15, 12.10 (2 x s, 1H), 10.96 (2 x s, 1H), 9.47 (br, 2H), 7.64 (d, 1H), 7.39 (s, 1H), 6.29 (m, 2H), 4.40 (br, 1H), 3.32, 3.23 (2 x d, 2H), 2.65, 2.44 (2 x m, 1H), 1.90 (m, 1H), 1.78 (m, 2H), 1.50 (m, 5H), 0.94 (m, 1H) ppm. m.p.: 152-160°C.

### N-(4-(2,4-Dihydroxyphenyl)thiazol-2-yl)-cyclohexancarboxamid:

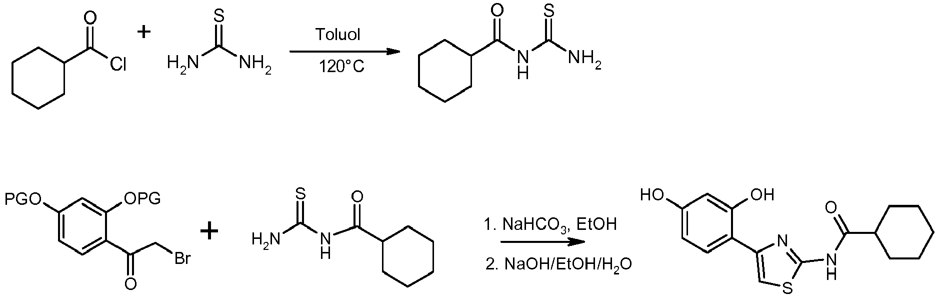

52 g (0.68 mol) Thioharnstoff wurden in Toluol (500 ml) vorgelegt und 50 g (0.34 mol) Cyclohexanoylchlorid zugetropft. Die Reaktionslösung wurde 3 Stunden unter Rückfluss gekocht, wobei 2 Phasen entstehen. Die obere Phase wurde abdekantiert und abgekühlt. Die ausgefallenen Kristalle wurden abgesaugt, mit Toluol gewaschen und aus Methanol umkristallisiert. Ausbeute: 35 g. ¹H NMR (DMSO-D₆): 10.98 (bs, 1H), 9.65 (bs, 1H), 9.32 (bs, 1H), 2.49 (t, 1H), 1.75 (m, 4H), 1.61 (m, 1H), 1.18 (m, 5H) ppm.

92 g (265 mmol) 2-Brom-2',4'- bis-methoxycarbonyloxy-acetophenon wurden mit 49.4 g (265 mmol) N-Cyclohexanoylthioharnstoff und 34 g (397 mmol) NaHCO₃ in 900 ml Ethanol unter Rückfluss für 0.5h gekocht. Die Reaktionslösung wurde abgekühlt und mit 37 g (930 mmol) NaOH in 300 ml Wasser versetzt. Nach 30 Min. Rühren bei Raumtemperatur wurde die Reaktionslösung mit 300 ml Wasser aufgenommen und mit 2N HCl neutralisiert. Das Ethanol wurde weitgehend am Rotationsverdampfer entfernt. Der entstandene Niederschlag wurde abfiltriert und aus Ethanol/Wasser umkristallisiert. Es wurden 70 g Thiazol erhalten. ¹H NMR (DMSO-D₆): 12.14 (bs, 1H), 11.00 (bs, 1H), 9.48 (bs, 1H), 7.64 (1 arom. H), 7.39 (s, 1H), 6.30 (2 arom. H), 2.49 (m, 1H), 1.84 (m, 2H), 1.76 (m, 2H), 1.65 (m, 1H), 1.42 (m, 2H), 1.25 (m, 3H) ppm. m.p.: 262-266°C.

### N-(4-(2,4-dihydroxyphenyl)thiazol-2-yl)-2-(4-(hydroxymethyl)phenyl)acetamid:

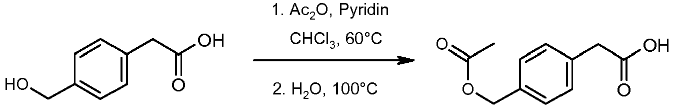

### Durchführung analog Literatur.

BANYU Pharmaceutical Co., Ltd., EP2072519 A1, 2009
Ausbeute: 76%, ¹H NMR (DMSO-D₆): 12.31 (bs, 1H), 7.26 (m, 4H), 5.05 (s, 2H), 3.57 (s, 2H), 2.05 (s, 3H) ppm; 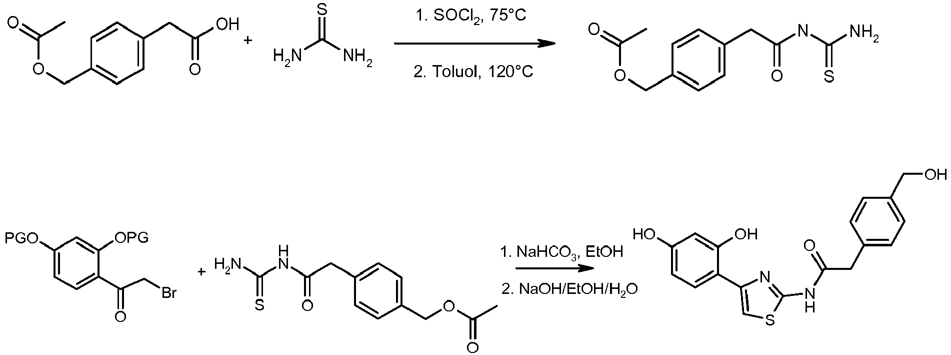 3.7 g (18 mmol) 4-Acetoxymethylphenylessigsäure wurden in 40 ml Thionylchlorid 2 h unter Rückfluss erhitzt. Nach Entfernen des überschüssigen Thionylchlorids im Vakuum wurde der Rückstand in 70 ml Toluol aufgenommen und 2.7 g (36 mmol) Thioharnstoff zugegeben. Die Reaktionslösung wurde 3 Stunden unter Rückfluss gekocht und anschließend wurde das Lösungsmittel im Vakuum entfernt. Die Reinigung erfolgte mittels Säulenchromatographie mit Cyclohexan/Essigester 1/1 an Kieselgel. Ausbeute: 2.7 g. ¹H NMR (DMSO-D₆): 11.29 (bs, 1H), 9.55 (bs, 1H), 9.40 (bs, 1H), 7.30 (m, 4H), 5.04 (s, 2H), 3.71 (s, 2H), 2.05 (s, 3H) ppm;
3.5 g (10 mmol) 2-Brom-2',4'- bis-methoxycarbonyloxy-acetophenon wurden mit 2.7 g (10 mmol) N-[2-(4-Acetoxymethylphenyl)acetyl]thioharnstoff und 1.3 g (15 mmol) NaHCO₃ in 50 ml Ethanol unter Rückfluss für 0.5h gekocht. Die Reaktionslösung wurde abgekühlt und mit 4.0 g (0.1 mol) NaOH in 20 ml Wasser versetzt. Nach 2 h Rühren bei 60°C wurde die Reaktionslösung in 100 ml Wasser aufgenommen und mit 2N HCl auf pH=3 eingestellt. Der entstandene Niederschlag wurde abfiltriert und aus Ethanol/Wasser umkristallisiert. Es wurden 1.3 g Thiazol erhalten. ¹H NMR (DMSO-D₆): 12.44 (s, 1H), 10.80 (s, 1H), 9.48 (s, 1H), 7.66 (d, 1H), 7.41 (s, 1H), 7.29 (m, 4H), 6.32 (m, 2H), 5.13 (t, 1H), 4.47 (d, 2H), 3.77 (s, 2H) ppm. m.p.: 254-256°C.

Kosmetische oder dermatologische Zubereitungen mit einem Gehalt an Alkylamidothiazolen bzw. deren Verwendung zur Behandlung und/oder Prophylaxe unerwünschter Hautpigmentierung, sind ebenfalls vorteilhafte Verkörperungen der vorliegenden Erfindung.

Vorteilhaft ist es insbesondere, wenn solche Zubereitungen 0,000001 bis 10 Gew.-%, insbesondere 0,0001 bis 3 Gew.-%, ganz besonders 0,001 bis 1 Gew.-% an einem oder mehreren der erfindungsgemäß verwendeten Alkylamidothiazolen enthalten, bezogen auf das Gesamtgewicht der Zubereitung.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-ÖI-in-Wasser (W/O/W), ein Gel, einen festen Stift, eine Salbe oder auch ein Aerosol darstellen. Es ist auch erfindungsgemäß vorteilhaft, die erfindungsgemäß verwendeten Substanzen und/oder deren Derivate in verkapselter Form darzureichen, z.B. in Kollagenmatrices und anderen üblichen Verkapselungsmaterialien, z.B. als Celluloseverkapselungen, in Gelatine oder liposomal verkapselt.

Es ist auch möglich und vorteilhaft im Sinne der vorliegenden Erfindung, die erfindungsgemäß verwendeten Substanzen und/oder deren Derivate in wäßrige Systeme bzw. Tensidzubereitungen zur Reinigung der Haut und der Haare einzufügen.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Siliconöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Die nachfolgenden Beispiele sollen die vorliegende Erfindung verdeutlichen, ohne sie einzuschränken. Alle Mengenangaben, Anteile und Prozentanteile sind, soweit nicht anders angegeben, Gewichtsprozente, auf das Gewicht und die Gesamtmenge bzw. auf das Gesamtgewicht der Zubereitungen bezogen.

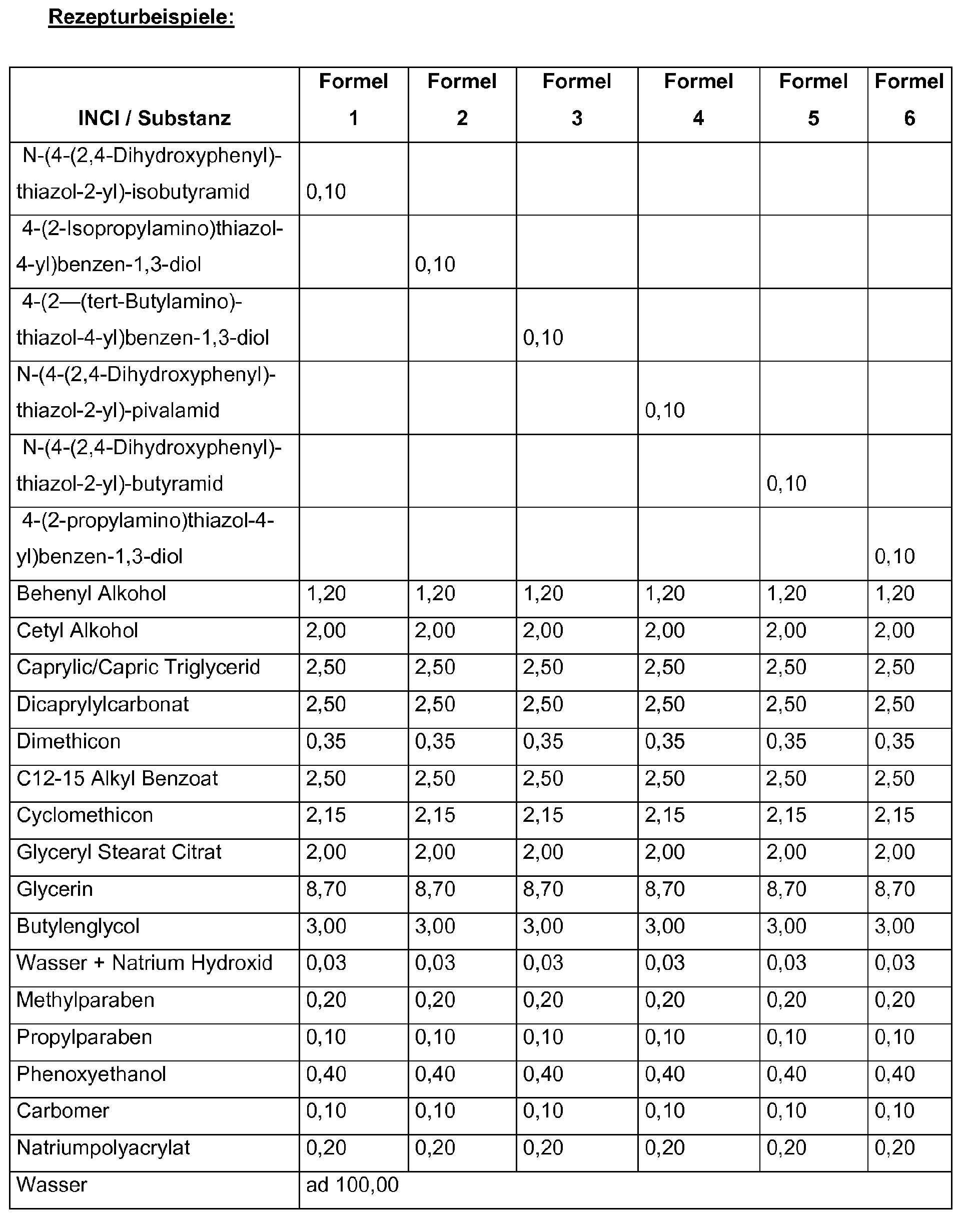

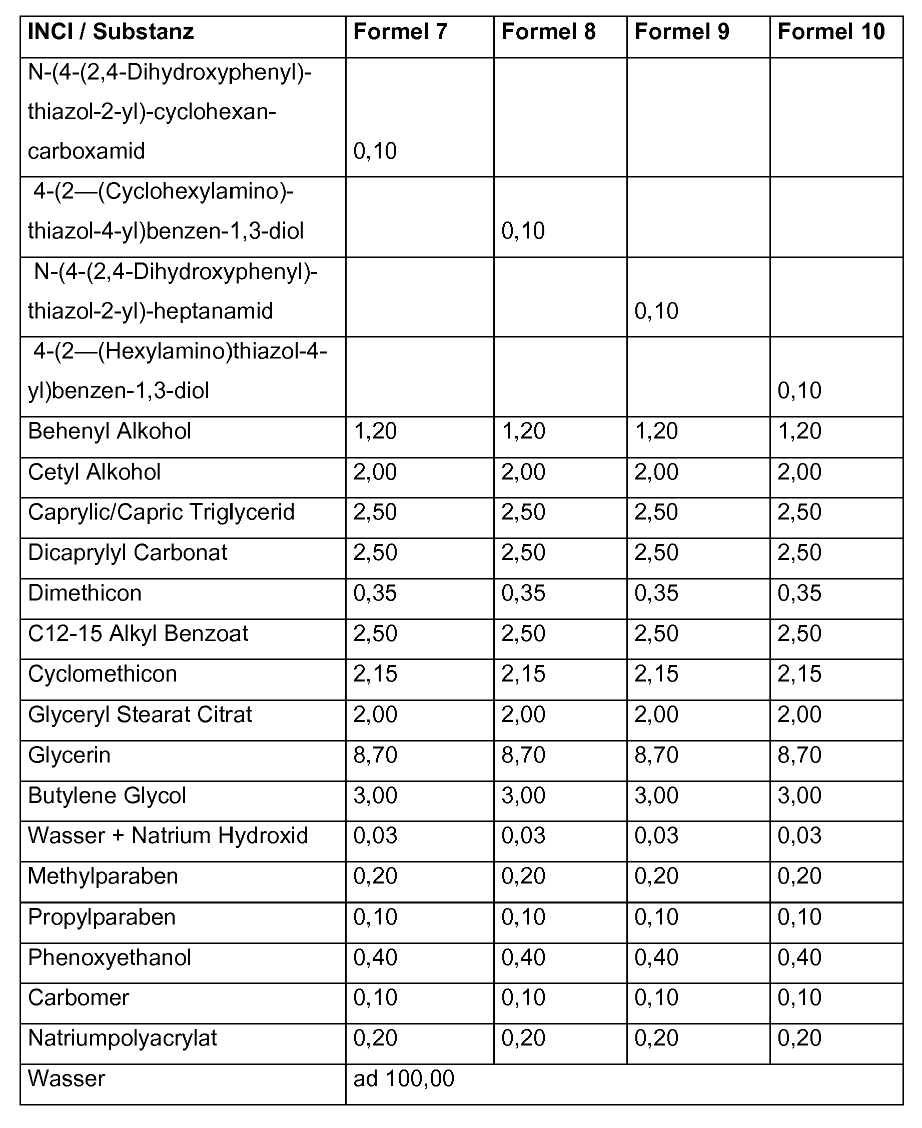

## Patentansprüche

1. Alkylamidothiazole der allgemeinen Formel 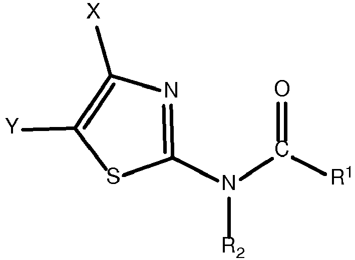 bei welcher
R¹, R², X und Y können unterschiedlich, teilweise gleich oder völlig gleich sein und unabhängig voneinander bedeuten können:
**X =** 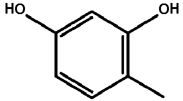
**Y** = H
**R₁ =** -C₁-C₂₄-Alkyl (linear und verzweigt), -C₁-C₂₄-Alkenyl (linear und verzweigt), -C₁-C₈-Cycloalkyl, -C₁-C₈-Cycloalkyl-Alkylhydroxy, -C₁-C₂₄ Alkylhydroxy (linear und verzweigt), -C₁-C₂₄ Alkylamin (linear und verzweigt), -C₁-C₂₄-Alkylaryl-Alkyl-Hydroxy (linear und verzweigt), -C₁-C₂₄-Alkylheteroaryl (linear und verzweigt), -C₁-C₂₄-Alkyl-O-C₁-C₂₄-Alkyl (linear und verzweigt), -C₁-C₂₄ Alky-Morpholino, -C₁-C₂₄ Alky-Piperidino,-C₁-C₂₄ Alky-Piperazino, -C₁-C₂₄ Alky-Piperazino-N-Alkyl bedeutet,
**R₂** = H.
wobei die Thiazole sowohl als freie Base wie auch als kosmetisch und dermatologisch verwendbare Salze vorliegen können.

2. Kosmetische oder dermatologische Zubereitungen mit einem Gehalt an einem oder mehreren Alkylamidothiazolen, wie sie in Anspruch 1 definiert sind.

3. Zubereitungen nach Anspruch 2, **dadurch gekennzeichnet, dass** das oder die Thiazole als Halogenid, Carbonat, Ascorbat, , Sulfat, Acetat und/oder Phosphat vorliegen.

4. Zubereitungen nach Anspruch 2 oder 3, enthaltend 0,000001 bis 10 Gew.-%, insbesondere 0,0001 bis 3 Gew.-%, ganz besonders 0,001 bis 1 Gew.-% an einem oder mehreren der in Anspruch 1 definierten Alkylamidothiazole, bezogen auf das Gesamtgewicht der Zubereitung.

5. Alkylamidothiazole, oder Zubereitungen, eines oder mehrere solcher Alkylamidothiazole enthaltend, nach Anspruch 1 zur Verwendung zur kosmetischen oder dermatologischen Behandlung und/oder Prophylaxe unerwünschter Hautpigmentierung.

## Claims

1. Alkylamidothiazoles of the general formula 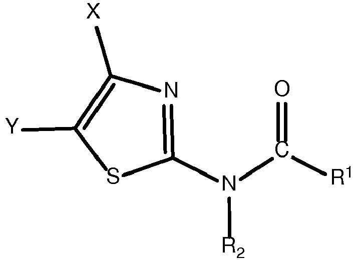 in which
R¹, R², X and Y may be different, partially identical or entirely identical, and independently of one another may have the following meanings:
**X =** 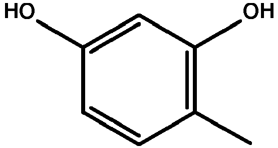
**Y** = H
**R₁** = -C₁-C₂₄ alkyl (linear and branched), -C₁-C₂₄ alkenyl (linear and branched), -C₁-C₈ cycloalkyl, -C₁-C₈ cycloalkyl-alkylhydroxy, -C₁-C₂₄ alkylhydroxy (linear and branched), -C₁-C₂₄ alkylamine (linear and branched), -C₁-C₂₄ alkylaryl (linear and branched), -C₁-C₂₄ alkylaryl-alkyl-hydroxy (linear and branched), -C₁-C₂₄ alkylheteroaryl (linear and branched), -C₁-C₂₄ alkyl-O-C₁-C₂₄ alkyl (linear and branched), -C₁-C₂₄ alkyl-morpholino, -C₁-C₂₄ alkyl-piperidino, -C₁-C₂₄ alkyl-piperazino, -C₁-C₂₄ alkyl-piperazino-N-alkyl,
**R₂** = H, wherein the thiazoles may be present both as free base and as cosmetically and dermatologically acceptable salts.

2. Cosmetic or dermatological formulations having a content of one or more alkylamidothiazoles as defined in Claim 1.

3. Formulations according to Claim 2, **characterized in that** the thiazole(s) are present as halide, carbonate, ascorbate, sulfate, acetate and/or phosphate.

4. Formulations according to Claim 2 or 3, comprising 0.000001 to 10 wt%, especially 0.0001 to 3 wt%, most particularly 0.001 to 1 wt% of one or more of the alkylamidothiazoles defined in Claim 1, based on the total weight of the formulation.

5. Alkylamidothiazoles, or formulations comprising one or more such alkylamidothiazoles, according to Claim 1, for use for the cosmetic or dermatological treatment and/or prophylaxis of undesired skin pigmentation.

## Revendications

1. Alkylamidothiazoles de formule générale 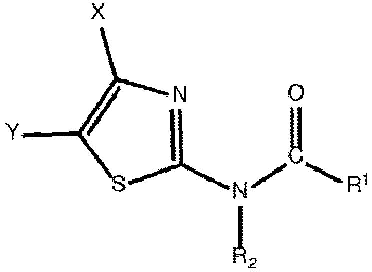 dans laquelle
R¹, R², X et Y peuvent être différents, partiellement identiques ou complètement identiques, et peuvent signifier indépendamment les uns des autres :
X = 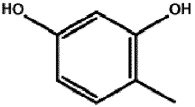
Y = H
R₁ = alkyle en C₁-C₂₄ (linéaire et ramifié), alcényle en C₁-C₂₄ (linéaire et ramifié), cycloalkyle en C₁-C₈, cycloalkyle en C₁-C₈-alkylhydroxy, alkylhydroxy en C₁-C₂₄ (linéaire et ramifié), alkylamine en C₁-C₂₄ (linéaire et ramifiée), alkylaryl-alkyl-hydroxy en C₁-C₂₄ (linéaire et ramifié), alkylhétéroaryle en C₁-C₂₄ (linéaire et ramifié), alkyle en C₁-C₂₄-O-alkyle en C₁-C₂₄ (linéaire et ramifié), alkyl-morpholino en C₁-C₂₄, alkyl-pipéridino en C₁-C₂₄, alkyl-pipérazino en C₁-C₂₄, alkyl-pipérazino-N-alkyle en C₁-C₂₄,
R₂ = H,
les thiazoles pouvant se présenter aussi bien sous la forme de la base libre que sous la forme de sels cosmétiquement et dermatologiquement utilisables.

2. Préparations cosmétiques ou dermatologiques ayant une teneur en un ou plusieurs alkylamidothiazoles, tels que définis dans la revendication 1.

3. Préparations selon la revendication 2, **caractérisées en ce que** le ou les thiazoles se présentent sous la forme d'un halogénure, d'un carbonate, d'un ascorbate, d'un sulfate, d'un acétate et/ou d'un phosphate.

4. Préparations selon la revendication 2 ou 3, contenant 0,000001 à 10 % en poids, notamment 0,0001 à 3 % en poids, tout particulièrement 0,001 à 1 % en poids d'un ou de plusieurs alkylamidothiazoles définis dans la revendication 1, par rapport au poids total de la préparation.

5. Alkylamidothiazoles ou préparations contenant un ou plusieurs tels alkylamidothiazoles selon la revendication 1, destinés à une utilisation pour le traitement cosmétique ou dermatologique et/ou la prophylaxie d'une pigmentation indésirable de la peau.
